# EUROPEAN PATENT SPECIFICATION

(11) **EP 4 433 483 B1**
(45) Date of publication and mention of the grant of the patent: **14.01.2026**
(21) Application number: 22839535.6
(22) Date of filing: 15.11.2022
(51) Int. Cl.: C07D 493/10, A61K 8/49, C11D 3/50, A61Q 13/00

(54) **FRAGRANCE SUBSTANCES AND FORMULATION CONTAINING THEM**
DUFTSTOFFE UND FORMULIERUNG, DIE SIE ENTHÄLT
SUBSTANCES PARFUMANTES ET FORMULATION LES CONTENANT

(30) Priority: 19.11.2021 US 202163281197 P
(43) Date of publication of application: 25.09.2024
(73) Proprietor: International Flavors & Fragrances Inc., New York, NY 10019 (US)
(72) Inventor: LEVORSE, JR., Anthony T., Union Beach, New Jersey 07735 (US); WEISS, Richard A., Union Beach, New Jersey 07735 (US); NARULA, Anubhav P.S., Union Beach, New Jersey 07735 (US); ANCONA, Lauren A., Union Beach, New Jersey 07735 (US); MONTELEONE, Michael G., Union Beach, New Jersey 07735 (US)
(74) Representative: Barker Brettell LLP
(86) International application number: PCT/US2022/049888
(87) International publication number: WO 2023/091387

(56) References cited:
- WO-A1-2015/036402
- WO-A1-2022/058021
- GULA MICHAEL J. ET AL: "Syntheses of 10-(carboxymethyl)-trans-decal-2-one", THE JOURNAL OF ORGANIC CHEMISTRY, vol. 45, no. 5, 1 February 1980 (1980-02-01) - 1 January 1980 (1980-01-01), pages 805 - 809, XP093020638, ISSN: 0022-3263, DOI: 10.1021/jo01293a009
- CAMBIE RC ET AL: "Chemistry of the Podocarpaceae. LXIV. Studies in the ambergris odorant field", AUSTRALIAN JOURNAL OF CHEMISTRY, vol. 35, no. 8, 1 January 1982 (1982-01-01), AU, pages 1699, XP093020644, ISSN: 0004-9425, DOI: 10.1071/CH9821699
- API A M ET AL: "RIFM fragrance ingredient safety assessment, lactoscatone, CAS Registry Number 21280-29-5", FOOD AND CHEMICAL TOXICOLOGY, vol. 127, 29 January 2019 (2019-01-29) - 29 January 2019 (2019-01-29), XP085663263, ISSN: 0278-6915, DOI: 10.1016/J.FCT.2019.01.022

## Description

### Field of the Invention

The present invention relates to new chemical entities and the incorporation and use of chemical entities as fragrance materials.

### Background of the Invention

There is an ongoing need in the fragrance industry to provide new chemicals to give perfumers and other persons the ability to create new fragrances for perfumes, colognes and personal care products. Those with skill in the art appreciate how differences in the chemical structure of the molecule can result in significant differences in the odor, notes and characteristics of a molecule. These variations and the ongoing need to discover and use the new chemicals in the development of new fragrances allow the perfumers to apply the new compounds in creating new fragrances.

WO2015036402A1 discloses compounds of Formula X, where R = -H, -methyl, ethyl, O-methyl or O-ethyl, R¹= -H, -methyl, ethyl, O-methyl or O-ethyl and n = 1 or 2. The disclosure also relates to the uses of said compounds as fragrance products, to preferred mixtures, to consumer goods containing said compounds and to the production of the compounds.

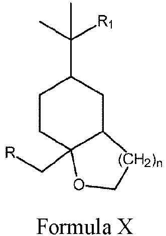

### Summary of the Invention

The present invention provides novel compounds. The present invention also provides compounds having unexpected advantageous use in enhancing, improving or modifying the fragrance of perfumes, colognes, toilet water, fabric care products, personal products and the like. The scope of the invention is defined by the appended claims.

More specifically, the present invention relates to a novel compound represented by Formula I set forth below: 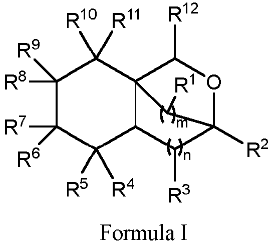
wherein R¹, R², R³, R⁴, R⁵, R⁶, R⁷, R⁸, R⁹, R¹⁰, R¹¹ and R¹² are each independently selected from the group consisting of H and CH_{3;} and
m and n are each an integer of 1 or 2, provided that m and n are not identical and wherein the compound is selected from the group consisting of:
2,6,6,9-tetramethyloctahydro-2*H*-2,4a-(epoxymethano)naphthalene;
1,3,8,8-tetramethyloctahydro-1*H*-3,9a-methanobenzo[c]oxepine;
6,6,9-trimethyloctahydro-2*H*-2,4a-(epoxymethano)naphthalene;
1,8,8-trimethyloctahydro-1*H*-3,9a-methanobenzo[c]oxepine;
4,6,6,9-tetramethyloctahydro-2*H*-2,4a-(epoxymethano)naphthalene;
1,8,8,10-tetramethyloctahydro-1*H*-3,9a-methanobenzo[c]oxepine;
2,4,6,6,9-pentamethyloctahydro-2*H*-2,4a-(epoxymethano)naphthalene;
1,3,5,8,8-pentamethyloctahydro-1*H*-3,9a-methanobenzo[c]oxepine;
2,3,6,6,9-pentamethyloctahydro-2*H*-2,4a-(epoxymethano)naphthalene;
1,3,4,8,8-pentamethyloctahydro-1*H*-3,9a-methanobenzo[c]oxepine;
1,3,8,8,10-pentamethyloctahydro-1*H*-3,9a-methanobenzo[c]oxepine;
1,2,6,6,9-pentamethyloctahydro-2*H*-2,4a-(epoxymethano)naphthalene; and
a mixture thereof.

Another embodiment of the present invention relates to a fragrance formulation comprising an olfactory acceptable amount of a compound of 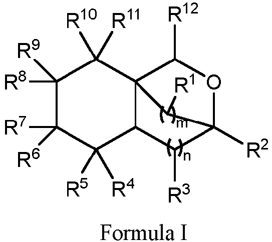 wherein R¹, R², R³, R⁴, R⁵, R⁶, R⁷, R⁸, R⁹, R¹⁰, R¹¹ and R¹² are each independently selected from the group consisting of H and CH_{3;} and
m and n are each an integer of 1 or 2, provided that m and n are not identical.

Another embodiment of the present invention relates to a fragrance product comprising the fragrance formulation provided above.

Another embodiment of the present invention relates to a method of improving, enhancing or modifying a fragrance formulation through the addition of an olfactory acceptable amount of a compound of 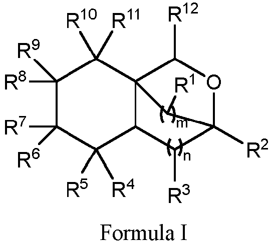 wherein R¹, R², R³, R⁴, R⁵, R⁶, R⁷, R⁸, R⁹, R¹⁰, R¹¹ and R¹² are each independently selected from the group consisting of H and CH_{3;} and
m and n are each an integer of 1 or 2, provided that m and n are not identical.

Another embodiment of the present invention relates to a method of counteracting malodor in an air space or a substrate comprising the step of introducing in the air space or the substrate a malodor counteracting effective amount of the fragrance formulation provided above.

These and other embodiments of the present invention will be apparent by reading the following specification.

### Detailed Description of the Invention

Formula I of the present invention can be illustrated, for example, by following examples:
2,6,6,9-tetramethyloctahydro-2*H*-2,4a-(epoxymethano)naphthalene;
1,3,8,8-tetramethyloctahydro-1*H*-3,9a-methanobenzo[c]oxepine;
6,6,9-trimethyloctahydro-2*H*-2,4a-(epoxymethano)naphthalene;
1,8,8-trimethyloctahydro-1*H*-3,9a-methanobenzo[c]oxepine;
4,6,6,9-tetramethyloctahydro-2*H*-2,4a-(epoxymethano)naphthalene;
1,8,8,10-tetramethyloctahydro-1*H*-3,9a-methanobenzo[c]oxepine;
2,4,6,6,9-pentamethyloctahydro-2*H*-2,4a-(epoxymethano)naphthalene;
1,3,5,8,8-pentamethyloctahydro-1*H*-3,9a-methanobenzo[c]oxepine;
2,3,6,6,9-pentamethyloctahydro-2*H*-2,4a-(epoxymethano)naphthalene;
1,3,4,8,8-pentamethyloctahydro-1*H*-3,9a-methanobenzo[c]oxepine;
1,3,8,8,10-pentamethyloctahydro-1*H*-3,9a-methanobenzo[c]oxepine;
1,2,6,6,9-pentamethyloctahydro-2*H*-2,4a-(epoxymethano)naphthalene; and
a mixture thereof.

The compounds of the present invention may be prepared via a general scheme depicted as follows: 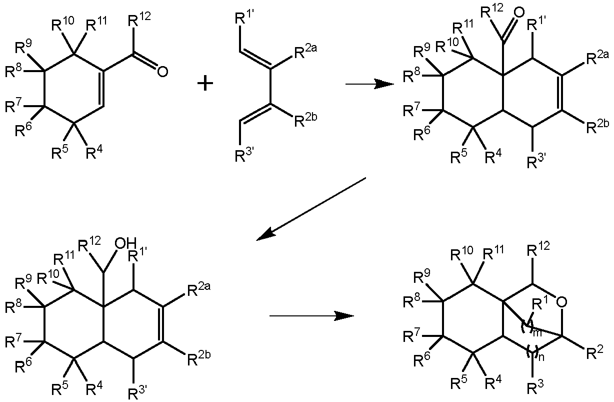 wherein R¹, R², R³, R⁴, R⁵, R⁶, R⁷, R⁸, R⁹, R¹⁰, R¹¹, R¹², m and n are defined as above; and wherein R¹, R^{2a}, R^{2b} and R^{3'} are each independently selected from the group consisting of H and CH_{3.}

Those with skill in the art will recognize that the compounds of the present invention and positional isomers thereof contain a number of chiral centers, thereby providing a number of stereoisomers. It is intended herein that the compounds of the present invention include isomeric mixtures as well as individual isomers that may be separated using techniques known to those having skill in the art. Suitable techniques include chromatography such as high performance liquid chromatography, referred to as HPLC, particularly silica gel chromatograph, and gas chromatography trapping known as GC trapping. Yet, commercial versions of such products are mostly offered as mixtures. The term "an isomer" is understood to mean a compound that has the same molecular formula but a different molecular structure.

The term "a compound" is understood to mean one or more of the compounds represented by formulas as described herein. The preparation of the compounds of the present invention is detailed in the Examples. Materials were purchased from Aldrich Chemical Company unless noted otherwise.

The use of the compounds of the present invention is widely applicable in current perfumery products, including the preparation of perfumes and colognes, the perfuming of personal care products such as soaps, shower gels, and hair care products, fabric care products, air fresheners, and cosmetic preparations. The present invention can also be used to perfume cleaning agents, such as, but not limited to detergents, dishwashing materials, scrubbing compositions, window cleaners and the like.

In these preparations, the compounds of the present invention can be used alone or in combination with other perfuming compositions, solvents, adjuvants and the like. The nature and variety of the other ingredients that can also be employed are known to those with skill in the art. Many types of fragrances can be employed in the present invention, the only limitation being the compatibility with the other components being employed. Suitable fragrances include but are not limited to fruits such as almond, apple, cherry, grape, pear, pineapple, orange, strawberry, raspberry; musk, flower scents such as lavender-like, rose-like, iris-like, carnation-like. Other pleasant scents include herbal and woodland scents derived from pine, spruce and other forest smells. Fragrances may also be derived from various oils, such as essential oils, or from plant materials such as peppermint, spearmint and the like.

A list of suitable fragrances is provided in US Pat. No. 4,534,891. Another source of suitable fragrances is found in Perfumes, Cosmetics and Soaps, Second Edition, edited by W. A. Poucher, 1959. Among the fragrances provided in this treatise are acacia, cassis, chypre, cyclamen, fern, gardenia, hawthorn, heliotrope, honeysuckle, hyacinth, jasmine, lilac, lily, magnolia, mimosa, narcissus, freshly-cut hay, orange blossom, orchid, reseda, sweet pea, trefle, tuberose, vanilla, violet, wallflower, and the like.

The compounds of the present invention can be used in combination with a complementary fragrance compound. The term "complementary fragrance compound" as used herein is defined as a fragrance compound selected from the group consisting of 2-[(4-methylphenyl)methylene]-heptanal (Acalea), iso-amyl oxyacetic acid allylester (Allyl Amyl Glycolate), 4,4,10,10,11,12,12-heptamethyl-3-oxatricyclo[7.3.0.0<2,6>]dodecane (Amber Xtreme), (3,3-dimethylcyclohexyl)ethyl ethyl propane-1,3-dioate (Applelide), (E/Z)-1-ethoxy-1-decene (Arctical), 2-ethyl-4-(2,2,3-trimethyl-3-cyclo-penten-1-yl)-2-buten-1-ol (Bacdanol), 2-methyl-3-[(1,7,7-trimethylbicyclo[2.2.1]hept-2-yl)oxy] exo-1-propanol (Bornafix), 1,2,3,5,6,7-hexahydro-1,1,2,3,3-pentamethyl-4*H*-inden-4-one (Cashmeran), trimethylcyclopentenylmethyloxabicyclooctane (Cassiffix), 1,1-dimethoxy-3,7-dimethyl-2,6-octadiene (Citral DMA), 3,7-dimethyl-6-octen-1-ol (Citronellol), 3A,4,5,6,7,7A-hexahydro-4,7-methano-1*H*-inden-5/6-yl acetate (Cyclacet), 3A,4,5,6,7,7A-hexahydro-4,7-methano-*1H*-inden-5/6-yl propinoate (Cyclaprop), 3A,4,5,6,7,7A-hexahydro-4,7-methano-1G-inden-5/6-yl butyrate (Cyclobutanate), 1-(2,6,6-trimethyl-3-cyclohexen-1-yl)-2-buten-1-one (Delta Damascone), 3-(4-ethylphenyl)-2,2-dimethyl propanenitrile (Fleuranil), 3-(O/P-ethylphenyl) 2,2-dimethyl propionaldehyde (Floralozone), tetrahydro-4-methyl-2-(2-methylpropyl)-2*H*-pyran-4-ol (Floriffol), 1,3,4,6,7,8-hexahydro-4,6,6,7,8,8-hexamethylcyclopenta-gamma-2-benzopyran (Galaxolide), 1-(5,5-dimethyl-1-cyclohexen-1-yl)pent-4-en-1-one (Galbascone), E/Z-3,7-dimethyl-2,6-octadien-1-yl acetate (Geranyl Acetate), α-methyl-1,3-benzodioxole-5-propanal (Helional), 1-(2,6,6-trimethyl-2-cyclohexen-1-yl)-1,6-heptadien-3-one (Hexalon), (Z)-3-hexenyl-2-hydroxybenzoate (Hexenyl Salicylate, CIS-3), 4-(2,6,6-trimethyl-2-cyclohexen-1-yl)-3-buten-2-one (Ionone α), 1-(1,2,3,4,5,6,7,8-octahydro-2,3,8,8-tetramethyl-2-naphthalenyl)-ethan-1-one (Iso E Super), methyl 3-oxo-2-pentylcyclopentaneacetate (Kharismal), 2,2,4-trimethyl-4-phenyl-butanenitrile (Khusinil), 3,4,5,6,6-pentamethylhept-3-en-2-one (Koavone), 3/4-(4-hydroxy-4-methyl-pentyl) cyclohexene-1-carboxaldehyde (Lyral), 3-methyl-4-(2,6,6-trimethyl-2-cyclohexen-1-yl)-3-buten-2-one (Methyl Ionone y), 1-(2,6,6-trimethyl-2-cyclohexen-1-yl) pent-1-en-3-one (Methyl Ionone α Extra, Methyl Ionone N), 3-methyl-4-phenylbutan-2-ol (Muguesia), cyclopentadec-4-en-1-one (Musk Z4), 3,3,4,5,5-pentamethyl-11,13-dioxatricyclo[7.4.0.0<2,6>]tridec-2(6)-ene (Nebulone), 3,7-dimethyl-2,6-octadien-1-yl acetate (Neryl Acetate), 3,7-dimethyl-1,3,6-octatriene (Ocimene), ortho-tolylethanol (Peomosa), 3-methyl-5-phenylpentanol (Phenoxanol), 1-methyl-4-(4-methyl-3-pentenyl) cyclohex-3-ene-1-carboxaldehyde (Precyclemone B), 4-methyl-8-methylene-2-adamantanol (Prismantol), 2-ethyl-4-(2,2,3-trimethyl-3-cyclopenten-1-yl)-2-buten-1-ol (Sanjinol), 2-methyl-4-(2,2,3-trimethyl-3-cyclopenten-1-yl)-2-buten-1-ol (Santaliff), 3-[cis-4-(2-methylpropyl)cyclohexyl]propanal (Starfleur), Terpineol, 2,4-dimethyl-3-cyclohexene-1-carboxaldehyde (Triplal), decahydro-2,6,6,7,8,8-hexamethyl-2*H*-indeno[4,5-B]furan (Trisamber), 2-tert-butylcyclohexyl acetate (Verdox), (3E)-4-methyldec-3-en-5-one (Veridian), 4-tert-butylcyclohexyL acetate (Vertenex), acetyl cedrene (Vertofix), 3,6/4,6-dimethylcyclohex-3-ene-1-carboxaldehyde (Vertoliff) and (3Z)-1-[(2-methyl-2-propenyl)oxy]-3-hexene (Vivaldie).

The term "alkyl" means a linear or branched saturated monovalent hydrocarbon. Examples include, for example, but not limited to, methyl, ethyl, propyl, 2-propyl, butyl (including all isomeric forms), pentyl (including all isomeric forms), hexyl (including all isomeric forms), and the like. The term "alkenyl" means a linear or branched unsaturated, aliphatic hydrocarbon containing at least one carbon-carbon double bond. The term "alkylene" refers to bivalent alkyl. Examples include, for example, but not limited to, -CH₂-, -CH₂CH₂-, -CH₂CH₂CH₂-, -CH₂(CH₃)CH₂-, -CH₂CH₂CH₂CH₂-, and the like.

The term "cycloalkyl" means a saturated monocyclic, fused bicyclic, or fused tricyclic, monovalent hydrocarbon radical of three to fourteen carbon ring atoms. Unless otherwise stated, the valency of the group may be located on any atom of any ring within the radical, valency rules permitting. More specifically, examples include, for example, but not limited to, cyclopropyl, cyclobutyl, cyclopentyl, cyclohexyl, cycloheptyl, decahydronaphthyl (e.g., decahydronaphth-1-yl, decahydronaphth-2-yl, and the like), norbornyl, and the like. The cycloalkyl ring is unsubstituted or may be substituted with one or more ring system substituents which may be the same or different, and are as defined herein. The term "cycloalkenyl" means a one or more double bond-containing monocyclic, fused bicyclic, or fused tricyclic, monovalent hydrocarbon radical of three to fourteen carbon ring atoms. Unless otherwise stated, the valency of the group may be located on any atom of any ring within the radical, valency rules permitting. More specifically, examples include, for example, but not limited to, cyclopropenyl, cyclobutenyl, cyclopentenyl, cyclohexenyl, and the like. The cycloalkenyl ring is unsubstituted or may be substituted with one or more ring system substituents which may be the same or different, and are as defined herein.

The term "ring system substituent" means a substituent attached to an aromatic or nonaromatic ring system, which, for example, replaces an available hydrogen on the ring system. Ring system substituents may be the same or different, each being independently being, *e.g.,-*C(=NH)(NH₂), -NHC(=NH)(NH₂), alkyl, alkenyl, alkynyl, alkoxy, acyl, alkylcarbonylamino, carboxy, carboxyalkyl, alkoxycarbonyl, alkoxycarbonylalkyl, cyano, nitro, alkylthio, halo, haloalkyl, haloalkoxy, amino, alkylamino, dialkylamino, aminocarbonyl, alkyl aminocarbonyl, dialkylaminocarbonyl, alkyl sulfonyl, cycloalkylsulfonyl, alkylsulfonylamino, alkylaminosulfonyl, haloalkylamino, oxo, hydroxy, hydroxyalkyl, hydroxyalkyloxy, hydroxyalkyloxyalkyl, alkoxyalkyloxyalkyl, aryl, heteroaryl, cycloalkyl, cycloalkylamino, cycloalkyloxy, heteroaralkyloxy, aminoalkyl, aminoalkyloxy, alkoxyalkyl, alkoxyalkylcarbonyl, alkoxyalkyloxy, haloalkoxyalkyl, aryloxyalkyl, heteroaryloxyalkyl, heterocycloalkyl, heterocycloalkyloxyalkyl, heterocycloalkylalkyl, heterocycloalkylalkyloxy, or heterocycloalkyloxy. "Ring system substituent" may also mean a single moiety that simultaneously replaces two available hydrogens on two adjacent carbon atoms (one H on each carbon) on a ring system. Examples of such moieties are methylenedioxy and ethylenedioxy.

The terms "fragrance formulation", "fragrance composition", and "perfume composition" mean the same and refer to a consumer composition that is a mixture of compounds including, for example, alcohols, aldehydes, ketones, esters, ethers, lactones, nitriles, natural oils, synthetic oils, and mercaptans, which are admixed so that the combined odors of the individual components produce a pleasant or desired fragrance. The fragrance formulation of the present invention is a consumer composition comprising a compound of the present invention. The fragrance formulation of the present invention comprises a compound of the present invention and further a complementary fragrance compound as defined above.

The term "fragrance product" means a consumer product containing a fragrance ingredient that adds fragrance or masks malodor. Fragrance products may include, for example, perfumes, colognes, bar soaps, liquid soaps, shower gels, foam baths, cosmetics, skin care products such as creams, lotions and shaving products, hair care products for shampooing, rinsing, conditioning, bleaching, coloring, dyeing and styling, deodorants and antiperspirants, feminine care products such as tampons and feminine napkins, baby care products such as diapers, bibs and wipes, family care products such as bath tissues, facial tissues, paper handkerchiefs or paper towels, fabric products such as fabric softeners and fresheners, air care products such as air fresheners and fragrance delivery systems, cosmetic preparations, cleaning agents and disinfectants such as detergents, dishwashing materials, scrubbing compositions, glass and metal cleaners such as window cleaners, countertop cleaners, floor and carpet cleaners, toilet cleaners and bleach additives, washing agents such as all-purpose, heavy duty, and hand washing or fine fabric washing agents including laundry detergents and rinse additives, dental and oral hygiene products such as toothpastes, tooth gels, dental flosses, denture cleansers, denture adhesives, dentifrices, tooth whitening and mouthwashes, health care and nutritional products and food products such as snack and beverage products. The fragrance product of the present invention is a consumer product that contains a compound of the present invention. The fragrance product of the present invention contains a compound of the present invention and further a complementary fragrance compound as defined above.

The term "improving" in the phrase "improving, enhancing or modifying a fragrance formulation" is understood to mean raising the fragrance formulation to a more desirable character. The term "enhancing" is understood to mean making the fragrance formulation greater in effectiveness or providing the fragrance formulation with an improved character. The term "modifying" is understood to mean providing the fragrance formulation with a change in character.

The term "olfactory acceptable amount" is understood to mean the amount of a compound in a fragrance formulation, wherein the compound will contribute its individual olfactory characteristics. However, the olfactory effect of the fragrance formulation will be the sum of effect of each of the fragrance ingredients. Thus, the compound of the present invention can be used to improve or enhance the aroma characteristics of the fragrance formulation, or by modifying the olfactory reaction contributed by other ingredients in the formulation. The olfactory acceptable amount may vary depending on many factors including other ingredients, their relative amounts and the olfactory effect that is desired.

The amount of the compounds of the present invention employed in a fragrance formulation varies from about 0.005 to about 70 weight percent, preferably from 0.005 to about 50 weight percent, more preferably from about 0.5 to about 25 weight percent, and even more preferably from about 1 to about 10 weight percent. Those with skill in the art will be able to employ the desired amount to provide desired fragrance effect and intensity. In addition to the compounds of the present invention, other materials can also be used in conjunction with the fragrance formulation to encapsulate and/or deliver the fragrance. Some well-known materials are, for example, but not limited to, polymers, oligomers, other non-polymers such as surfactants, emulsifiers, lipids including fats, waxes and phospholipids, organic oils, mineral oils, petrolatum, natural oils, perfume fixatives, fibers, starches, sugars and solid surface materials such as zeolite and silica. Some preferred polymers include polyacrylate, polyurea, polyurethane, polyacrylamide, polyester, polyether, polyamide, poly(acrylate-co-acrylamide), starch, silica, gelatin and gum Arabic, alginate, chitosan, polylactide, poly(melamine-formaldehyde), poly(urea-formaldehyde), or a combination thereof.

When used in a fragrance formulation these ingredients provide additional notes to make a fragrance formulation more desirable and noticeable, and add the perception of value. The odor qualities found in these materials assist in beautifying and enhancing the finished accord as well as improving the performance of the other materials in the fragrance.

In addition, the compounds of the present invention are also surprisingly found to provide superior ingredient performance and possess unexpected advantages in malodor counteracting applications such as body perspiration, environmental odor such as mold and mildew, bathroom, and etc. The compounds of the present invention substantially eliminate the perception of malodors and/or prevent the formation of such malodors, thus, can be utilized with a vast number of functional products.

Examples of the functional products are provided herein to illustrate the various aspects of the present invention. However, they do not intend to limit the scope of the present invention. The functional products may include, for example, a conventional room freshener (or deodorant) composition such as room freshener sprays, an aerosol or other spray, fragrance diffusers, a wick or other liquid system, or a solid, for instance candles or a wax base as in pomanders and plastics, powders as in sachets or dry sprays or gels, as in solid gel sticks, clothes deodorants as applied by washing machine applications such as in detergents, powders, liquids, whiteners or fabric softeners, fabric refreshers, linen sprays, closet blocks, closet aerosol sprays, or clothes storage areas or in dry cleaning to overcome residual solvent notes on clothes, bathroom accessories such as paper towels, bathroom tissues, sanitary napkins, towellets, disposable wash cloths, disposable diapers, and diaper pail deodorants, cleansers such as disinfectants and toilet bowl cleaners, cosmetic products such as antiperspirant and deodorants, general body deodorants in the form of powders, aerosols, liquids or solid, or hair care products such as hair sprays, conditioners, rinses, hair colors and dyes, permanent waves, depilatories, hair straighteners, hair groom applications such as pomade, creams and lotions, medicated hair care products containing such ingredients as selenium sulphide, coal tar or salicylates, or shampoos, or foot care products such as foot powders, liquids or colognes, after shaves and body lotions, or soaps and synthetic detergents such as bars, liquids, foams or powders, odor control such as during manufacturing processes, such as in the textile finishing industry and the printing industry (inks and paper), effluent control such as in processes involved in pulping, stock yard and meat processings, sewage treatment, garbage bags, or garbage disposal, or in product odor control as in textile finished goods, rubber finished goods or car fresheners, agricultural and pet care products such as dog and hen house effluents and domestic animal and pet care products such as deodorants, shampoo or cleaning agents, or animal litter material and in large scale closed air systems such as auditoria, and subways and transport systems.

Thus, it will be seen that the composition of the invention is usually one in which the malodor counteractant is present together with a carrier by means of which or from which the malodor counteractant can be introduced into air space wherein the malodor is present, or a substrate on which the malodor has deposited. For example, the carrier can be an aerosol propellant such as a chlorofluoro-methane, or a solid such as a wax, plastics material, rubber, inert powder or gel. In a wick-type air freshener, the carrier is a substantially odorless liquid of low volatility. In several applications, a composition of the invention contains a surface active agent or a disinfectant, while in others, the malodor counteractant is present on a fibrous substrate. In many compositions of the invention there is also present a fragrance component which imparts a fragrance to the composition. The fragrances stated above can all be employed.

Malodor counteracting effective amount is understood to mean the amount of the inventive malodor counteractant employed in a functional product that is organoleptically effective to abate a given malodor while reducing the combined intensity of the odor level, wherein the given malodor is present in air space or has deposited on a substrate. The exact amount of malodor counteractant agent employed may vary depending upon the type of malodor counteractant, the type of the carrier employed, and the level of malodor counteractancy desired. In general, the amount of malodor counteractant agent present is the ordinary dosage required to obtain the desired result. Such dosage is known to the skilled practitioner in the art. In a preferred embodiment, when used in conjunction with malodorous solid or liquid functional products, e.g., soap and detergent, the compounds of the present invention may be present in an amount ranging from about 0.005 to about 50 weight percent, preferably from about 0.01 to about 20 weight percent, and more preferably from about 0.05 to about 5 weight percent, and when used in conjunction with malodorous gaseous functional products, the compounds of the present invention may be present in an amount ranging from about 0.1 to 10 mg per cubic meter of air.

The following are provided as specific embodiments of the present invention. The scope of the invention is defined by the claims. As used herein all percentages are weight percent unless otherwise noted, ppm is understood to stand for parts per million, L is understood to be liter, mL is understood to be milliliter, g is understood to be gram, Kg is understood to be kilogram, mol is understood to be mole, mmol is understood to be millimole, psig is understood to be pound-force per square inch gauge, and mmHg be millimeters (mm) of mercury (Hg). IFF as used in the examples is understood to mean International Flavors & Fragrances Inc., New York, NY, USA.

### EXAMPLE I

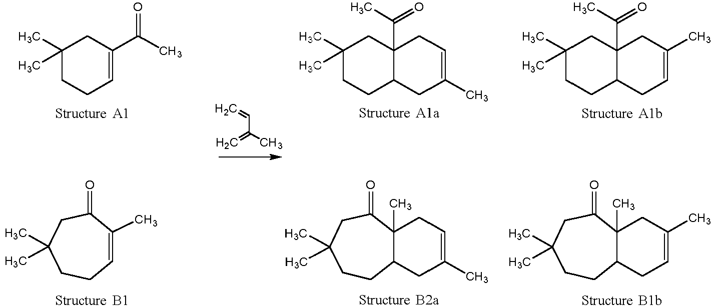

**Preparation of the Ketones:** A mixture of 1-((5,5)-dimethylcyclohex-1-en-lyl)ethan-1-one (Structure A1) and 2,6,6-trimethylcyclohept-2-en-1-one (Structure B1) further containing 1-((3,3)-dimethylcyclohex-1-en-lyl)ethan-1-one was prepared from dehydrolinalool according to known methods (e.g., U.S. Patent No. 4,147,672; Ansari, et al. (1973) Tetrahedron 29: 1559-1564) (450 g, 2.96 mol) and charged into a 3-L reaction vessel together with methylene chloride (500 g). The reaction was cooled to 15-20 °C. Solid aluminum trichloride (25.6 g, 0.19 mol) was added over 30 minutes while the temperature was maintained at 20-25 'C. Isoprene (261 g, 3.8 mol) was subsequently added over 6 hours while the temperature was allowed to rise to 35 °C. The reaction mass was quenched with wet ice, transferred to a separatory funnel, and washed with brine (i.e., saturated sodium chloride solution) (500 mL) followed by aqueous caustic (5%, 500 mL). The organic layer was removed and distilled to afford ketones of 1-(3,3,7-trimethyl-1,3,4,5,8,8a-hexahydronaphthalen-4a(2*H*)-yl)ethan-1-one (Structure A1a) (36%), 1-(3,3,6-trimethyl-1,3,4,5,8,8a-hexahydronaphthalen-4a(2*H*)-yl)ethan-1-one (Structure A1b) (36%), 2,4a,7,7-tetramethyl-1,4,4a,6,7,8,9,9a-octahydro-5*H*-benzo[7]annulen-5-one (Structure B1a) (4%), and 3,4a,7,7-tetramethyl-1,4,4a,6,7,8,9,9a-octahydro-5*H-*benzo[7]annulen-5-one (Structure B1b) (4%) (338 g). The ketone mixture had a boiling point of 140-142 °C at 2 mmHg.
1-(3,3,7 or 3,3,6-Trimethyl-1,3,4,5,8,8a-hexahydronaphthalen-4a(2*H*)-yl)ethan-1-one (Structure A1a and Structure A1b)
¹H NMR (500 MHz, CDCl₃) δ: 5.17-5.22 (m, 1H), 2.21-2.28 (m, 1H), 2.10 (s, 3H), 1.57-1.59 (m, 3H), 1.28-2.16 (m, 9H), 1.11-1.18 (m, 1H), 0.93 (s, 3H), 0.80 (s, 3H)
2,4a,7,7 or 3,4a,7,7-Tetramethyl-1,4,4a,6,7,8,9,9a-octahydro-5*H*-benzo[7]annulen-5-one (Structure B1a and Structure B1b)
¹H NMR (500 MHz, CDCl₃) δ: 5.23-5.28 (m, 1H), 2.89 (d, J=10.7 Hz, 1H), 2.31-2.41 (m, 1H), 1.28-2.16 (m, 8H), 1.93 (d, J=10.7 Hz, 1H), 1.60 (s, 3H), 1.00 (s, 3H), 0.97 (s, 3H), 0.82 (s, 3H)

The obtained ketone mixture was described as having green, woody, herbal, floral and cassis notes.

### EXAMPLE II

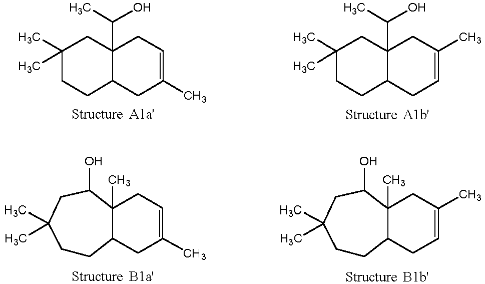

**Preparation of the Alcohols:** A 2-L reaction vessel was charged with Red-Al^{®} (279 g, 1.65 mol) and heated to 50-55 °C. The ketone products of EXAMPLE I (338 g) was fed into the reaction vessel over 3 hours. The reaction mass was quenched with ethyl acetate (50 g) and heated to 75-80 °C. Aqueous caustic soda (25%, 500 mL) was added slowly to the reaction mass. The reaction was aged for 2 hours and cooled to an ambient temperature. The reaction mass was transferred to a separatory funnel. The organic layer was removed, retreated with aqueous caustic soda (25%, 500 mL) at 75-80 °C, and washed with brine (500 mL). The organic layer was removed and distilled to afford alcohols of 1-(3,3,7-trimethyl-1,3,4,5,8,8a-hexahydronaphthalen-4a(2*H*)-yl)ethan-1-ol (Structure A1a'), 1-(3,3,6-trimethyl-1,3,4,5,8,8a-hexahydronaphthalen-4a(2*H*)-yl)ethan-1-ol (Structure A1b') (with a weight ratio of Structure A1a' and Structure A1b' of 1:1), 2,4a,7,7-tetramethyl-4,4a,5,6,7,8,9,9a-octahydro-1*H-*benzo[7]annulen-5-ol (Structure B1a'), and 3,4a,7,7-tetramethyl-4,4a,5,6,7,8,9,9a-octahydro-1*H*-benzo[7]annulen-5-ol (Structure B1b') (310 g). Each product contained further isomers. The alcohol mixture had a boiling point of 132-134 °C at 2 mmHg.
1-(3,3,7 or 3,3,6-Trimethyl-1,3,4,5,8,8a-hexahydronaphthalen-4a(2*H*)-yl)ethan-1-ol (Structure A1a' and Structure A1b')
¹H NMR (500 MHz, CDCl₃) δ: 5.13-5.22 (m, 2H), 3.81 (q, J=6.5 Hz, 1H), 3.76 (q, J=6.5 Hz, 1H), 1.00-2.43 (m, 24H), 1.59 (s, 6H), 1.10 (d, J=6.5 Hz, 3H), 1.05 (d, J=6.5 Hz, 3H), 1.00 (s, 6H), 0.91 (s, 6H)
2,4a,7,7 or 3,4a,7,7-Tetramethyl-4,4a,5,6,7,8,9,9a-octahydro-1*H*-benzo[7]annulen-5-ol (Structure B1a' and Structure B1b')
¹H NMR (500 MHz, CDCl₃) δ: 5.25-5.30 (m, 1H), 3.59 (d, J=9.7 Hz, 1H), 1.00-2.45 (m, 12H), 1.65 (s, 3H), 0.94 (m, 3H), 0.93 (m, 3H), 0.87 (m, 3H)

The obtained alcohol mixture was described as having leather, moss, and cresolic notes.

### EXAMPLE III

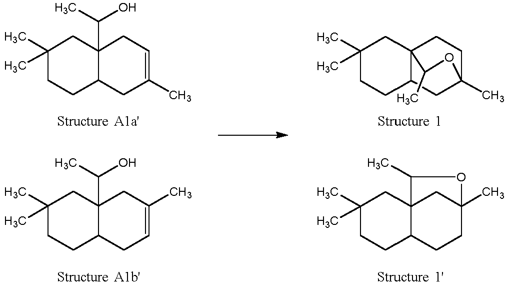

**Preparation of 2,6,6,9-Tetramethyloctahydro-2*H*-2,4a-(epoxymethano)naphthalene (Structure 1) and 1,3,8,8-tetramethyloctahydro-1*H*-3,9a-methanobenzo[c]oxepine (Structure 1'):** A 2-L reaction vessel was charged with the alcohol mixture of EXAMPLE II (310 g, 1.4 mol), toluene (500 mL), and methanesulfonic acid (24 g, 0.25 mol) and heated to 75-80 °C. The reaction was aged for 10 hours and cooled to an ambient temperature. The reaction mass was transferred to a separatory funnel and washed with saturated aqueous sodium carbonate solution (300 mL) followed by brine (300 mL). The organic layer was removed and distilled to afford 2,6,6,9-tetramethyloctahydro-2*H*-2,4a-(epoxymethano)naphthalene (Structure 1) and 1,3,8,8-tetramethyloctahydro-1*H*-3,9a-methanobenzo[c]oxepine (Structure 1') (217 g) with a weight ratio of 1:1. Structure B1a' and Structure B1b' were left unrecovered in the distillation. The products had a boiling point of 118-119 °C at 2 mmHg.

¹H NMR (500 MHz, CDCl₃) δ: 3.76 (q, J=6.3 Hz, 1H), 3.39 (q, J=6.3 Hz, 1H), 2.17-2.25 (m, 1H), 1.97-2.07 (m, 1H), 1.86-1.94 (m, 1H), 1.70-1.83 (m, 2H), 0.74-1.67 (m, 21H), 1.09 (d, J=6.3 Hz, 3H), 1.04 (d, J=6.3 Hz, 3H), 1.02 (s, 6H), 0.95 (s, 3H), 0.93 (s, 3H), 0.84 (s, 6H)

The obtained Structure 1 and Structure 1' mixture was described as having woody, camphor and sweet notes.

### EXAMPLE IV

**Preparation of 1-(3,3-Dimethyl-1,3,4,5,8,8a-hexahydronaphthalen-4a(2H)-yl)ethan-1-one (Structure A2) and 4a,7,7-Trimethyl-1,4,4a,6,7,8,9,9a-octahydro-5*H*-benzo[7]annulen-5-one (Structure B2):** Structure A2 (85%) and Structure B2 (13%) were similarly prepared using butadiene according to EXAMPLE I.
1-(3,3-Dimethyl-1,3,4,5,8,8a-hexahydronaphthalen-4a(2*H*)-yl)ethan-1-one (Structure A2)
¹H NMR (500 MHz, CDCl₃) δ: 5.47-5.60 (m, 2H), 2.22 (m, 1H), 1.98-2.17 (m, 2H), 2.10 (s, 3H), 1.86-2.09 (m, 2H), 1.43 (m, 2H), 1.31-1.70 (m, 2H), 1.08-1.47 (m, 2H), 0.94 (s, 3H), 0.81 (s, 3H)
4a,7,7-Trimethyl-1,4,4a,6,7,8,9,9a-octahydro-5*H*-benzo[7]annulen-5-one (Structure B2)
¹H NMR (500 MHz, CDCl₃) δ: 5.44-5.63 (m, 2H), 2.89 (d, J=10.9 Hz, 1H), 1.10-2.45 (m, 10H), 0.99 (s, 3H), 0.94 (s, 3H), 0.82 (s, 3H)

The obtained Structure A2 and Structure B2 mixture was described as having woody and slight gourmand notes.

**Preparation of 1-(3,3-Dimethyl-1,3,4,5,8,8a-hexahydronaphthalen-4a(2*H*)-yl)ethan-1-ol (Structure A2') and 4a,7,7-Trimethyl-1,4,4a,6,7,8,9,9a-octahydro-5*H*-benzo[7]annulen-5-ol (Structure B2'):** Structure A2' and Structure B2' were prepared from Structure A2 and Structure B2 according to EXAMPLE II.
1-(3,3-Dimethyl-1,3,4,5,8,8a-hexahydronaphthalen-4a(2*H*)-yl)ethan-1-ol (Structure A2')
¹H NMR (500 MHz, CDCl₃) δ: 5.44-5.66 (m, 2H), 3.85 (m, 1H), 1.49 (s, 1H), 1.20-2.50 (m, 6H), 1.37-1.85 (m, 1H), 1.01-1.48 (m, 4H), 1.07-1.15 (d, J=6.6 Hz, 3H), 1.03 (s, 3H), 0.93 (s, 3H)
4a,7,7-Trimethyl-1,4,4a,6,7,8,9,9a-octahydro-5*H*-benzo[7]annulen-5-ol (Structure B2')
¹H NMR (500 MHz, CDCl₃) δ: 5.43-5.67 (m, 2H), 3.62 (d, J=9.5 Hz, 1H), 1.04-2.52 (m, 12H), 0.97 (s, 3H), 0.96 (s, 3H), 0.91-0.94 (m, 3H).

The obtained Structure A2' and Structure B2' mixture was described as having woody and camphoraceous notes.

Pr**eparation of 6,6,9-Trimethyloctahydro-2*H*-2,4a-(epoxymethano)naphthalene (Structure 2) and 1,8,8-Trimethyloctahydro-1*H*-3,9a-methanobenzo[c]oxepine (Structure 2'):** Mixture of Structure 2 and Structure 2' with a weight ratio of 1:2 was prepared from Structure A2' according to EXAMPLE III.
¹H NMR (500 MHz, CDCl₃) δ: 4.26-4.35 (m, 1H), 3.57 (q, J=6.6 Hz, 1H), 1.98-2.15 (m, 2H). 1.53-1.79 (m, 3H), 1.07-1.52 (m, 7H), 1.20 (d, J=6.6 Hz, 3H), 0.92-0.99 (m, 4H), 0.91 (s, 3H).

The obtained Structure 2 and Structure 2' mixture was described as having ambery and woody notes.

### EXAMPLE V

**Preparation of 1-(3,3,5-Trimethyl-1,3,4,5,8,8a-hexahydronaphthalen-4a(2*H*)-yl)ethan-1-one (Structure A3) and 3,4a,7,7-Tetramethyl-1,4,4a,6,7,8,9,9a-octahydro-5*H-*benzo[7]annulen-5-one (Structure B3):** Structure A3 (83%) and Structure B3 (**13%**) were similarly prepared using piperylene according to EXAMPLE I.
1-(3,3,5-Trimethyl-1,3,4,5,8,8a-hexahydronaphthalen-4a(2*H*)-yl)ethan-1-one (Structure A3)
¹H NMR (400 MHz, CDCl₃) δ: 5.44-5.75 (m, 2H), 2.36-2.52 (m, 1H), 2.14 (s, 3H), 1.81-2.08 (m, 4H), 1.52-1.64 (m, 2H), 1.34-1.47 (m, 1H), 1.05-1.25 (m, 2H), 0.87 (s, 3H), 0.81 (s, 3H), 0.72 (d, J=7.0 Hz)
3,4a,7,7-Tetramethyl-1,4,4a,6,7,8,9,9a-octahydro-5*H*-benzo[7]annulen-5-one (Structure B3)
¹H NMR (400 MHz, CDCl₃) δ: 5.44-5.75 (m, 2H), 2.84 (d, J=11.5 Hz, 1H), 1.15-2.50 (m, 9H), 1.12 (d, J=7.3 Hz, 3H), 1.08 (s, 3H), 1.02 (s, 3H), 0.95 (s, 3H).

The obtained Structure A3 and Structure B3 mixture was described as having leather, woody, and camphoraceous notes.

**Preparation of 1-(3,3,5-Trimethyl-1,3,4,5,8,8a-hexahydronaphthalen-4a(2*H*)-yl)ethan-1-ol (Structure A3') and 3,4a,7,7-tetramethyl-1,4,4a,6,7,8,9,9a-octahydro-5*H*-benzo[7]annulen-5-ol (Structure B3'):** Structure A3' and Structure B3' were prepared from Structure A3 and Structure B3 according to EXAMPLE II.
1-(3,3,5-Trimethyl-1,3,4,5,8,8a-hexahydronaphthalen-4a(2*H*)-yl)ethan-1-ol (Structure A3')
¹H NMR (400 MHz, CDCl₃) δ: 5.23-5.75 (m, 2H), 3.98 (q, J=6.5 Hz, 1H), 3.66 (q, J=6.4 Hz, 1H), 2.75-2.95 (m, 1H), 2.52-2.64 (m, 1H), 2.20-2.32 (m, 1H), 2.06 (br s, 1H), 1.16-1.85 (m, 7H), 1.10-1.16 (m, 6H), 1.06 (s, 3H), 1.04 (s, 3H), 1.00-1.09 (m, 1H), 0.96 (s, 3H), 0.90 (s, 3H), 0.85-0.92 (m, 1H)
3,4a,7,7-tetramethyl-1,4,4a,6,7,8,9,9a-octahydro-5*H*-benzo[7]annulen-5-ol (Structure B3')
¹H NMR (400 MHz, CDCl₃) δ: 5.21-5.88 (m, 2H), 3.90-3.95 (m, 1H), 1.16-2.70 (m, 11H), 0.86-1.16 (m, 12H)

The obtained Structure A3' and Structure B3' mixture was described as having leather, woody, and violet notes.

**Preparation of 4,6,6,9-Tetramethyloctahydro-2H-2,4a-(epoxymethano)naphthalene (Structure 3) and 1,8,8,10-Tetramethyloctahydro-1*H*-3,9a-methanobenzo[c]oxepine (Structure 3'):** Mixture of Structure 3 and Structure 3' with a weight ratio of 5:2 was prepared from Structure A3' according to EXAMPLE III.

¹H NMR (500 MHz, CDCl₃) δ: 3.93-3.97 (m, 1H), 3.83-3.89 (m, 1H), 3.76 (q, J=6.6 Hz, 1H), 1.97-2.13 (m, 2H), 1.54-1.79 (m, 2H), 1.35-1.49 (m, 3H), 1.09-1.33 (m, 4H), 1.16 (d, J=6.6 Hz, 3H), 1.13 (d, J=6.8 Hz, 3H), 1.05 (d, J=7.0 Hz, 3H), 0.96-0.99 (m, 3H), 0.96-0.99 (m, 3H), 0.96 (s, 3H), 0.91 (s, 3H), 0.89-0.95 (m, 1H), 0.87 (s, 3H) 0.76 (d, J=14.0 Hz, 1H).

The obtained Structure 3 and Structure 3' mixture was described as having ambery, amber grey, and slightly woody notes.

### EXAMPLE VI

**Preparation of 2,4,6,6,9-Pentamethyloctahydro-2H-2,4a-(epoxymethano)naphthalene (Structure 4) and 1,3,5,8,8-Pentamethyloctahydro-1*H*-3,9a-methanobenzo[c]oxepine (Structure 4'):** Mixture of Structure 4 and Structure 4' with a weight ratio of 3:1 was similarly prepared using 2-methylpenta-1,3-diene according to EXAMPLE I-III.

### EXAMPLE VII

The fragrance formula exemplified as follows demonstrates that a fragrance formulation containing the Structure 1 and Structure 1' compounds with a weight ratio of 1:1 provided unexpected and superior synergistic fragrance effect. The accord exhibited woody, camphor and sweet notes.

| **Ingredients** | **Parts*** | |
|---|---|---|
| | - | + |
| AGRUNITRILE | 60.00 | 60.00 |
| ALD C-8 TOCO | 20.00 | 20.00 |
| ALD C-10 PRG STABILIFF | 18.00 | 18.00 |
| ALD C-9 TOCO | 2.80 | 2.80 |
| DIMETH BENZ CARB BUTY | 2.00 | 2.00 |
| HERBALIME | 100.00 | 100.00 |
| FRESCILE PPF | 0.30 | 0.30 |
| FENCHOL | 1.20 | 1.20 |
| GALBASCONE PRG TOCO 10% DPG | 1.80 | 1.80 |
| GERANIOL 980 PURE | 10.00 | 10.00 |
| HEXENOL CIS-3 | 1.00 | 1.00 |
| METH DH JASMONATE | 15.00 | 15.00 |
| LAVANDIN GROSSO OIL LMR CSM | 2.20 | 2.20 |
| LIME OIL WI TYPE TOCO | 7.00 | 7.00 |
| ALD C-11 MOA TOCO | 5.00 | 5.00 |
| MANGONE 1% DPG | 6.00 | 6.00 |
| METH HEPTENONE | 1.20 | 1.20 |
| OPALENE TOCO 10% IPM (MB) | 0.50 | 0.50 |
| PETITGRAIN OIL CSM | 1.50 | 1.50 |
| RHUBAFURAN TOCO | 0.40 | 0.40 |
| ALLYL CAPROATE | 4.50 | 4.50 |
| DAMASCONE ALPHA TOCO | 0.30 | 0.30 |
| HEXYL CINN ALD TBHQ | 30.00 | 30.00 |
| ALD C-12 MNA TOCO | 10.00 | 10.00 |

### EXAMPLE VII (CONTINUED)

*** "+" represents a Structure 1/ Structure 1' containing formula; and "-"represents a Structure 1/ Structure 1' non-containing formula.**

| **Ingredients** | **Parts*** | |
|---|---|---|
| | - | + |
| POMELENE TT 0.01PCT IPM | 14.00 | 14.00 |
| OXANE 50 PCT TEC 10% DPG | 0.70 | 0.70 |
| ETH BUTY | 0.60 | 0.60 |
| MANDARIN ALD 10 PCT CITR TOCO | 6.40 | 6.40 |
| SYLVONIC | 50.00 | 50.00 |
| CITRONELLAL NAT | 30.00 | 30.00 |
| HEXENYL CIS-3 ACET | 1.00 | 1.00 |
| LINALOOL SYN TOCO | 14.00 | 14.00 |
| LITSEA CUBEBA OIL FILT | 25.00 | 25.00 |
| CRISTALFIZZ | 3.00 | 3.00 |
| ORANGE OIL CP TOCO | 50.00 | 50.00 |
| CITRAL N | 100.00 | 100.00 |
| ETH-2-METH BUTY | 0.30 | 0.30 |
| GALBAZINE 1% DPG | 0.10 | 0.10 |
| VERTONIC | 0.20 | 0.20 |
| ALD AA TRIPLAL TOCO | 3.00 | 3.00 |
| ISO CYCLO CITRAL TOCO | 1.00 | 1.00 |
| DIPROPYLENE GLYCOL | 350.00 | 300.00 |
| **STRUCTURE 1/STRUCTURE 1'** | -- | 50.00 |
| **TOTAL** | 950.00 | 950.00 |

## Claims

1. A compound of 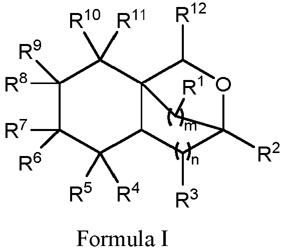
wherein R¹, R², R³, R⁴, R⁵, R⁶, R⁷, R⁸, R⁹, R¹⁰, R¹¹ and R¹² are each independently selected from the group consisting of H and CH_{3;} and
m and n are each an integer of 1 or 2, provided that m and n are not identical; and wherein the compound is selected from the group consisting of:
2,6,6,9-tetramethyloctahydro-2*H*-2,4a-(epoxymethano)naphthalene;
1,3,8,8-tetramethyloctahydro-1*H*-3,9a-methanobenzo[c]oxepine;
6,6,9-trimethyloctahydro-2*H*-2,4a-(epoxymethano)naphthalene;
1,8,8-trimethyloctahydro-1*H*-3,9a-methanobenzo[c]oxepine;
4,6,6,9-tetramethyloctahydro-2*H*-2,4a-(epoxymethano)naphthalene;
1,8,8,10-tetramethyloctahydro-1*H*-3,9a-methanobenzo[c]oxepine;
2,4,6,6,9-pentamethyloctahydro-2*H*-2,4a-(epoxymethano)naphthalene;
1,3,5,8,8-pentamethyloctahydro-1*H*-3,9a-methanobenzo[c]oxepine;
2,3,6,6,9-pentamethyloctahydro-2*H*-2,4a-(epoxymethano)naphthalene;
1,3,4,8,8-pentamethyloctahydro-1*H*-3,9a-methanobenzo[c]oxepine;
1,3,8,8,10-pentamethyloctahydro-1*H*-3,9a-methanobenzo[c]oxepine;
1,2,6,6,9-pentamethyloctahydro-2*H*-2,4a-(epoxymethano)naphthalene; and a mixture thereof.

2. A fragrance formulation containing an olfactory acceptable amount of a compound of 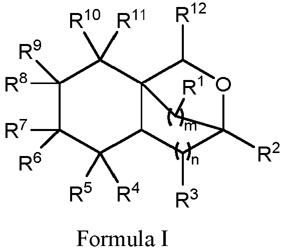
wherein R¹, R², R³, R⁴, R⁵, R⁶, R⁷, R⁸, R⁹, R¹⁰, R¹¹ and R¹² are each independently selected from the group consisting of H and CH_{3;} and
m and n are each an integer of 1 or 2, provided that m and n are not identical.

3. The fragrance formulation of claim 2, wherein the compound is selected from the group consisting of:
2,6,6,9-tetramethyloctahydro-2*H*-2,4a-(epoxymethano)naphthalene;
1,3,8,8-tetramethyloctahydro-1*H*-3,9a-methanobenzo[c]oxepine;
6,6,9-trimethyloctahydro-2*H*-2,4a-(epoxymethano)naphthalene;
1,8,8-trimethyloctahydro-1H-3,9a-methanobenzo[c]oxepine;
4,6,6,9-tetramethyloctahydro-2*H*-2,4a-(epoxymethano)naphthalene;
1,8,8,10-tetramethyloctahydro-1*H*-3,9a-methanobenzo[c]oxepine;
2,4,6,6,9-pentamethyloctahydro-2*H*-2,4a-(epoxymethano)naphthalene;
1,3,5,8,8-pentamethyloctahydro-1*H*-3,9a-methanobenzo[c]oxepine;
2,3,6,6,9-pentamethyloctahydro-2*H*-2,4a-(epoxymethano)naphthalene;
1,3,4,8,8-pentamethyloctahydro-1*H*-3,9a-methanobenzo[c]oxepine;
1,3,8,8,10-pentamethyloctahydro-1*H*-3,9a-methanobenzo[c]oxepine;
1,2,6,6,9-pentamethyloctahydro-2*H*-2,4a-(epoxymethano)naphthalene; and
a mixture thereof.

4. The fragrance formulation of claim 2 or of claim 3, wherein the olfactory acceptable amount is from:
(i) 0.005 to 50 weight percent of the fragrance formulation; or
(ii) 0.1 to 25 weight percent of the fragrance formulation; or
(iii) 0.5 to 10 weight percent of the fragrance formulation.

5. The fragrance formulation of claim 2 or of any preceding fragrance formulation claim further comprising a polymer.

6. The fragrance formulation of claim 5, wherein the polymer is selected from the group consisting of polyacrylate, polyurea, polyurethane, polyacrylamide, polyester, polyether, polyamide, poly(acrylate-co-acrylamide), starch, silica, gelatin and gum Arabic, alginate, chitosan, polylactide, poly(melamine-formaldehyde), poly(urea-formaldehyde) and a combination thereof.

7. A method of improving, enhancing or modifying a fragrance formulation through the addition of an olfactory acceptable amount of a compound of 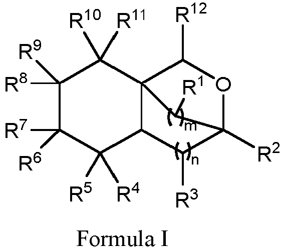
wherein R¹, R², R³, R⁴, R⁵, R⁶, R⁷, R⁸, R⁹, R¹⁰, R¹¹ and R¹² are each independently selected from the group consisting of H and CH_{3;} and
m and n are each an integer of 1 or 2, provided that m and n are not identical.

8. The method of claim 7, wherein the compound is selected from the group consisting of:
2,6,6,9-tetramethyloctahydro-2*H*-2,4a-(epoxymethano)naphthalene;
1,3,8,8-tetramethyloctahydro-1*H*-3,9a-methanobenzo[c]oxepine;
6,6,9-trimethyloctahydro-2*H*-2,4a-(epoxymethano)naphthalene;
1,8,8-trimethyloctahydro-1*H*-3,9a-methanobenzo[c]oxepine;
4,6,6,9-tetramethyloctahydro-2*H*-2,4a-(epoxymethano)naphthalene;
1,8,8,10-tetramethyloctahydro-1*H*-3,9a-methanobenzo[c]oxepine;
2,4,6,6,9-pentamethyloctahydro-2*H*-2,4a-(epoxymethano)naphthalene;
1,3,5,8,8-pentamethyloctahydro-1*H*-3,9a-methanobenzo[c]oxepine;
2,3,6,6,9-pentamethyloctahydro-2*H*-2,4a-(epoxymethano)naphthalene;
1,3,4,8,8-pentamethyloctahydro-1*H*-3,9a-methanobenzo[c]oxepine;
1,3,8,8,10-pentamethyloctahydro-1*H*-3,9a-methanobenzo[c]oxepine;
1,2,6,6,9-pentamethyloctahydro-2*H*-2,4a-(epoxymethano)naphthalene; and
a mixture thereof.

9. The method of claim 7 or claim 8, wherein the olfactory acceptable amount is from:
(i) 0.005 to 50 weight percent of the fragrance formulation; or
(ii) 0.1 to 25 weight percent of the fragrance formulation; or
(iii) 0.5 to 10 weight percent of the fragrance formulation.

10. A fragrance product containing the fragrance formulation of claim 2 or of any preceding fragrance formulation claim.

11. The fragrance product of claim 10, wherein the fragrance product is selected from the group consisting of a perfume, a cologne, toilet water, a cosmetic product, a personal care product, a fabric care product, a cleaning agent and an air freshener, a bar soap, a liquid soap, a shower gel, a foam bath, a cosmetic, a skin care product, a hair care product, a deodorant, an antiperspirant, a feminine care product, a baby care product, a family care product, a fabric product, an air care product, a fragrance delivery system, a cosmetic preparation, a disinfectant, a washing agent, a dental and oral hygiene product, a health care and nutritional product and a food product.

12. A method of counteracting malodor in an air space or a substrate comprising the step of introducing in the air space or the substrate a malodor counteracting effective amount of the fragrance formulation of claim 2 or of any preceding fragrance formulation claim.

## Patentansprüche

1. Verbindung der 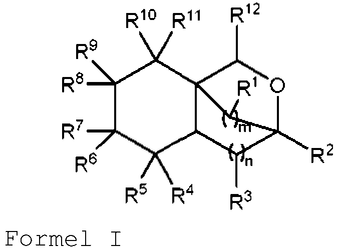
wobei R¹, R², R³, R⁴, R⁵, R⁶, R⁷, R⁸, R⁹, R¹⁰, R¹¹ und R¹² jeweils unabhängig aus der Gruppe bestehend aus H und CH₃ ausgewählt sind und
m und n jeweils eine ganze Zahl mit einem Wert von 1 oder
2 sind, mit der Maßgabe, dass m und n nicht identisch sind; und wobei die Verbindung ausgewählt ist aus der Gruppe bestehend aus:
2,6,6,9-Tetramethyloctahydro-2H-2,4a-(epoxymethano)naphthalin;
1,3,8,8-Tetramethyloctahydro-1H-3,9a-methanobenzo[c]oxepin;
6,6,9-Trimethyloctahydro-2H-2,4a-(epoxymethano)naphthalin;
1,8,8-Trimethyloctahydro-1H-3,9a-methanobenzo[c]oxepin;
4,6,6,9-Tetramethyloctahydro-2H-2,4a-(epoxymethano)naphthalin;
1,8,8,10-Tetramethyloctahydro-1H-3,9a-methanobenzo[c]oxepin;
2,4,6,6,9-Pentamethyloctahydro-2H-2,4a-(epoxymethano)naphthalin;
1,3,5,8,8-Pentamethyloctahydro-1H-3,9a-methanobenzo[c]oxepin;
2,3,6,6,9-Pentamethyloctahydro-2H-2,4a-(epoxymethano)naphthalin;
1,3,4,8,8-Pentamethyloctahydro-1H-3,9a-methanobenzo[c]oxepin;
1,3,8,8,10-Pentamethyloctahydro-1H-3,9a-methanobenzo[c]oxepin;
1,2,6,6,9-Pentamethyloctahydro-2H-2,4a-(epoxymethano)naphthalin und
einem Gemisch davon.

2. Duftstoffformulierung, enthaltend eine olfaktorisch annehmbare Menge einer Verbindung der 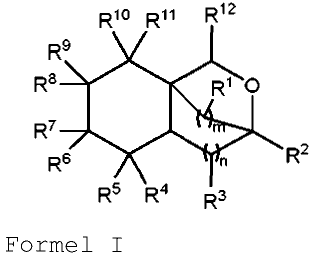
wobei R¹, R², R³, R⁴, R⁵, R⁶, R⁷, R⁸, R⁹, R¹⁰, R¹¹ und R¹² jeweils unabhängig aus der Gruppe bestehend aus H und CH₃ ausgewählt sind und
m und n jeweils eine ganze Zahl mit einem Wert von 1 oder 2 sind, mit der Maßgabe, dass m und n nicht identisch sind.

3. Duftstoffformulierung nach Anspruch 2, wobei die Verbindung ausgewählt ist aus der Gruppe bestehend aus:
2,6,6,9-Tetramethyloctahydro-2H-2,4a-(epoxymethano)naphthalin;
1,3,8,8-Tetramethyloctahydro-1H-3,9a-methanobenzo[c]oxepin;
6,6,9-Trimethyloctahydro-2H-2,4a-(epoxymethano)naphthalin;
1,8,8-Trimethyloctahydro-1H-3,9a-methanobenzo[c]oxepin;
4,6,6,9-Tetramethyloctahydro-2H-2,4a-(epoxymethano)naphthalin;
1,8,8,10-Tetramethyloctahydro-1H-3,9a-methanobenzo[c]oxepin;
2,4,6,6,9-Pentamethyloctahydro-2H-2,4a-(epoxymethano)naphthalin;
1,3,5,8,8-Pentamethyloctahydro-1H-3,9a-methanobenzo[c]oxepin;
2,3,6,6,9-Pentamethyloctahydro-2H-2,4a-(epoxymethano)naphthalin;
1,3,4,8,8-Pentamethyloctahydro-1H-3,9a-methanobenzo[c]oxepin;
1,3,8,8,10-Pentamethyloctahydro-1H-3,9a-methanobenzo[c]oxepin;
1,2,6,6,9-Pentamethyloctahydro-2H-2,4a-(epoxymethano)naphthalin und
einem Gemisch davon.

4. Duftstoffformulierung nach Anspruch 2 oder Anspruch 3, wobei die olfaktorisch annehmbare Menge Folgendes beträgt:
(i) 0,005 bis 50 Gew.-% der Duftstoffformulierung oder
(ii) 0,1 bis 25 Gew.-% der Duftstoffformulierung oder
(iii) 0,5 bis 10 Gew.-% der Duftstoffformulierung.

5. Duftstoffformulierung nach Anspruch 2 oder einem vorhergehenden Duftstoffformulierungsanspruch, ferner umfassend ein Polymer.

6. Duftstoffformulierung nach Anspruch 5, wobei das Polymer aus der Gruppe bestehend aus Polyacrylat, Polyharnstoff, Polyurethan, Polyacrylamid, Polyester, Polyether, Polyamid, Poly(acrylat-co-acrylamid), Stärke, Siliciumdioxid, Gelatine und Gummi arabicum, Alginat, Chitosan, Polylactid, Poly(melamin-formaldehyd), Poly(harnstoff-formaldehyd) und einer Kombination davon ausgewählt ist.

7. Verfahren zur Verbesserung, Verstärkung oder Modifizierung einer Duftstoffformulierung durch Zugabe einer olfaktorisch annehmbaren Menge einer Verbindung der 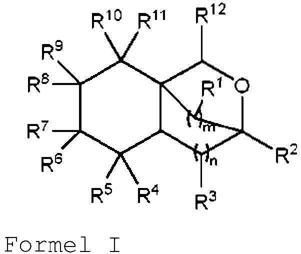
wobei R¹, R², R³, R⁴, R⁵, R⁶, R⁷, R⁸, R⁹, R¹⁰, R¹¹ und R¹² jeweils unabhängig aus der Gruppe bestehend aus H und CH₃ ausgewählt sind und
m und n jeweils eine ganze Zahl mit einem Wert von 1 oder 2 sind, mit der Maßgabe, dass m und n nicht identisch sind.

8. Verfahren nach Anspruch 7, wobei die Verbindung ausgewählt ist aus der Gruppe bestehend aus:
2,6,6,9-Tetramethyloctahydro-2H-2,4a-(epoxymethano)naphthalin;
1,3,8,8-Tetramethyloctahydro-1H-3,9a-methanobenzo[c]oxepin;
6,6,9-Trimethyloctahydro-2H-2,4a-(epoxymethano)naphthalin;
1,8,8-Trimethyloctahydro-1H-3,9a-methanobenzo[c]oxepin;
4,6,6,9-Tetramethyloctahydro-2H-2,4a-(epoxymethano)naphthalin;
1,8,8,10-Tetramethyloctahydro-1H-3,9a-methanobenzo[c]oxepin;
2,4,6,6,9-Pentamethyloctahydro-2H-2,4a-(epoxymethano)naphthalin;
1,3,5,8,8-Pentamethyloctahydro-1H-3,9a-methanobenzo[c]oxepin;
2,3,6,6,9-Pentamethyloctahydro-2H-2,4a-(epoxymethano)naphthalin;
1,3,4,8,8-Pentamethyloctahydro-1H-3,9a-methanobenzo[c]oxepin;
1,3,8,8,10-Pentamethyloctahydro-1H-3,9a-methanobenzo[c]oxepin;
1,2,6,6,9-Pentamethyloctahydro-2H-2,4a-(epoxymethano)naphthalin und
einem Gemisch davon.

9. Verfahren nach Anspruch 7 oder Anspruch 8, wobei die olfaktorisch annehmbare Menge Folgendes beträgt:
(i) 0,005 bis 50 Gew.-% der Duftstoffformulierung oder
(ii) 0,1 bis 25 Gew.-% der Duftstoffformulierung oder
(iii) 0,5 bis 10 Gew.-% der Duftstoffformulierung.

10. Duftstoffprodukt, enthaltend die Duftstoffformulierung nach Anspruch 2 oder einem vorhergehenden Duftstoffformulierungsanspruch.

11. Duftstoffprodukt nach Anspruch 10, wobei das Duftstoffprodukt aus der Gruppe bestehend aus einem Parfüm, einem Eau de Cologne, einem Toilettenwasser, einem kosmetischen Produkt, einem Körperpflegeprodukt, einem Textilpflegeprodukt, einem Reinigungsmittel und einem Lufterfrischer, einer Stückseife, einer Flüssigseife, einem Duschgel, einem Schaumbad, einem Kosmetikum, einem Hautpflegeprodukt, einem Haarpflegeprodukt, einem Deodorant, einer Antitranspirant, einem Damenhygieneprodukt, einem Säuglingspflegeprodukt, einem Familienpflegeprodukt, einem Textilprodukt, einem Luftpflegeprodukt, einem Duftstoffabgabesystem, einer kosmetischen Zubereitung, einem Desinfektionsmittel, einem Waschmittel, einem Zahn- und Mundhygieneprodukt, einem Gesundheits- und Ernährungsprodukt und einem Lebensmittelprodukt ausgewählt ist.

12. Verfahren zum Entgegenwirken von schlechtem Geruch in einem Luftraum oder einem Substrat, umfassend den Schritt des Eintragens einer zum Entgegenwirken von schlechtem Geruch wirksamen Menge der Duftstoffformulierung nach Anspruch 2 oder eines vorhergehenden Duftstoffformulierungsanspruchs in den Luftraum oder das Substrat.

## Revendications

1. Composé de 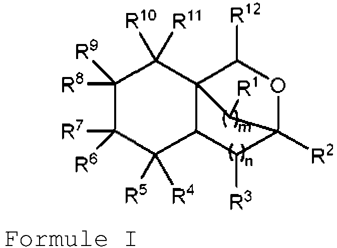
R¹, R², R³, R⁴, R⁵, R⁶, R⁷, R⁸, R⁹, R¹⁰, R¹¹ et R¹² étant chacun indépendamment choisis dans le groupe constitué par H et CH₃ ; et
m et n étant chacun un entier parmi 1 ou 2, à condition que m et n ne soient pas identiques ; et le composé étant choisi dans le groupe constitué par :
2,6,6,9-tétraméthyloctahydro-2H-2,4a-(époxyméthano)naphtalène ;
1,3,8,8-tétraméthyloctahydro-1H-3,9a-méthanobenzo[c]oxépine ;
6,6,9-triméthyloctahydro-2H-2,4a-(époxyméthano)naphtalène ;
1,8,8-triméthyloctahydro-1H-3,9a-méthanobenzo[c]oxépine ;
4,6,6,9-tétraméthyloctahydro-2H-2,4a-(époxyméthano)naphtalène ;
1,8,8,10-tétraméthyloctahydro-1H-3,9a-méthanobenzo[c]oxépine ;
2,4,6,6,9-pentaméthyloctahydro-2H-2,4a-(époxyméthano)naphtalène ;
1,3,5,8,8-pentaméthyloctahydro-1H-3,9a-méthanobenzo[c]oxépine ;
2,3,6,6,9-pentaméthyloctahydro-2H-2,4a-(époxyméthano)naphtalène ;
1,3,4,8,8-pentaméthyloctahydro-1H-3,9a-méthanobenzo[c]oxépine ;
1,3,8,8,10-pentaméthyloctahydro-1H-3,9a-méthanobenzo[c]oxépine ;
1,2,6,6,9-pentaméthyloctahydro-2H-2,4a-(époxyméthano)naphtalène ; et
un mélange correspondant.

2. Formulation de fragrance contenant une quantité olfactivement acceptable d'un composé de 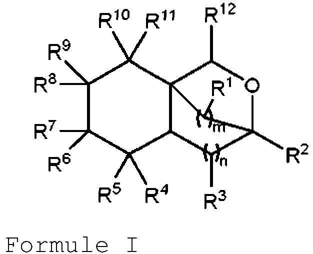
R¹, R², R³, R⁴, R⁵, R⁶, R⁷, R⁸, R⁹, R¹⁰, R¹¹ et R¹² étant chacun indépendamment choisis dans le groupe constitué par H et CH₃ ; et
m et n étant chacun un entier de 1 ou 2, à condition que m et n ne soient pas identiques.

3. Formulation de fragrance selon la revendication 2, le composé étant choisi dans le groupe constitué par :
2,6,6,9-tétraméthyloctahydro-2H-2,4a-(époxyméthano)naphtalène ;
1,3,8,8-tétraméthyloctahydro-1H-3,9a-méthanobenzo[c]oxépine ;
6,6,9-triméthyloctahydro-2H-2,4a-(époxyméthano)naphtalène ;
1,8,8-triméthyloctahydro-1H-3,9a-méthanobenzo[c]oxépine ;
4,6,6,9-tétraméthyloctahydro-2H-2,4a-(époxyméthano)naphtalène ;
1,8,8,10-tétraméthyloctahydro-1H-3,9a-méthanobenzo[c]oxépine ;
2,4,6,6,9-pentaméthyloctahydro-2H-2,4a-(époxyméthano)naphtalène ;
1,3,5,8,8-pentaméthyloctahydro-1H-3,9a-méthanobenzo[c]oxépine ;
2,3,6,6,9-pentaméthyloctahydro-2H-2,4a-(époxyméthano)naphtalène ;
1,3,4,8,8-pentaméthyloctahydro-1H-3,9a-méthanobenzo[c]oxépine ;
1,3,8,8,10-pentaméthyloctahydro-1H-3,9a-méthanobenzo[c]oxépine ;
1,2,6,6,9-pentaméthyloctahydro-2H-2,4a-(époxyméthano)naphtalène ; et
un mélange correspondant.

4. Formulation de fragrance selon la revendication 2 ou selon la revendication 3, dans laquelle la quantité olfactivement acceptable est de :
(i) 0,005 à 50 pour cent en poids de la formulation de fragrance ; ou
(ii) 0,1 à 25 pour cent en poids de la formulation de fragrance ; ou
(iii) 0,5 à 10 pour cent en poids de la formulation de fragrance.

5. Formulation de fragrance selon la revendication 2 ou selon l'une quelconque des revendications précédentes de formulation de fragrance comprenant en outre un polymère.

6. Formulation de fragrance selon la revendication 5, dans laquelle le polymère est choisi dans le groupe constitué par un polyacrylate, une polyurée, un polyuréthane, un polyacrylamide, un polyester, un polyéther, un polyamide, un poly(acrylate-co-acrylamide), un amidon, une silice, une gélatine et une gomme arabique, un alginate, un chitosane, un polylactide, un poly(mélamine-formaldéhyde), un poly(urée-formaldéhyde) et une combinaison de ceux-ci.

7. Procédé d'amélioration, d'augmentation ou de modification d'une formulation de fragrance par ajout d'une quantité olfactivement acceptable d'un composé de 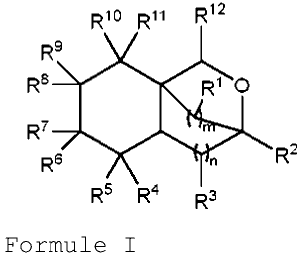
R¹, R², R³, R⁴, R⁵, R⁶, R⁷, R⁸, R⁹, R¹⁰, R¹¹ et R¹² étant chacun indépendamment choisis dans le groupe constitué par H et CH₃ ; et
m et n étant chacun un entier de 1 ou 2, à condition que m et n ne soient pas identiques.

8. Procédé selon la revendication 7, dans lequel le composé est choisi dans le groupe constitué par :
2,6,6,9-tétraméthyloctahydro-2H-2,4a-(époxyméthano)naphtalène ;
1,3,8,8-tétraméthyloctahydro-1H-3,9a-méthanobenzo[c]oxépine ;
6,6,9-triméthyloctahydro-2H-2,4a-(époxyméthano)naphtalène ;
1,8,8-triméthyloctahydro-1H-3,9a-méthanobenzo[c]oxépine ;
4,6,6,9-tétraméthyloctahydro-2H-2,4a-(époxyméthano)naphtalène ;
1,8,8,10-tétraméthyloctahydro-1H-3,9a-méthanobenzo[c]oxépine ;
2,4,6,6,9-pentaméthyloctahydro-2H-2,4a-(époxyméthano)naphtalène ;
1,3,5,8,8-pentaméthyloctahydro-1H-3,9a-méthanobenzo[c]oxépine ;
2,3,6,6,9-pentaméthyloctahydro-2H-2,4a-(époxyméthano)naphtalène ;
1,3,4,8,8-pentaméthyloctahydro-1H-3,9a-méthanobenzo[c]oxépine ;
1,3,8,8,10-pentaméthyloctahydro-1H-3,9a-méthanobenzo[c]oxépine ;
1,2,6,6,9-pentaméthyloctahydro-2H-2,4a-(époxyméthano)naphtalène ; et
un mélange correspondant.

9. Procédé selon la revendication 7 ou la revendication 8, dans lequel la quantité olfactive acceptable est de :
(i) 0,005 à 50 pour cent en poids de la formulation de fragrance ; ou
(ii) 0,1 à 25 pour cent en poids de la formulation de fragrance ; ou
(iii) 0,5 à 10 pour cent en poids de la formulation de fragrance.

10. Produit de fragrance contenant la formulation de fragrance selon la revendication 2 ou selon l'une quelconque des revendications précédentes de formulation de fragrance.

11. Produit de fragrance selon la revendication 10, dans lequel le produit de fragrance est choisi dans le groupe constitué par un parfum, une eau de Cologne, une eau de toilette, un produit cosmétique, un produit de soin personnel, un produit d'entretien de tissus, un agent de nettoyage et un assainissement d'air, un savon en barre, un savon liquide, un gel douche, un bain moussant, un produit cosmétique, un produit de soin de la peau, un produit de soin capillaire, un déodorant, un antitranspirant, un produit d'hygiène féminine, un produit de soin pour bébés, un produit de soin pour la famille, un produit pour tissus, un produit d'assainissement de l'air, un système de distribution de fragrance, une préparation cosmétique, un désinfectant, un agent de lavage, un produit d'hygiène dentaire et buccale, un produit de soins de santé et nutritionnel est un produit alimentaire.

12. Procédé pour lutte contre les mauvaises odeurs dans un espace d'air ou un substrat, comprenant l'étape d'introduction dans l'espace d'air ou le substrat d'une quantité efficace pour lutter contre les mauvaises odeurs de la formulation de fragrance selon la revendication 2 ou de l'une quelconque des revendications précédentes de formulation de fragrance.
